Europäisches Patentamt

(19) **European Patent Office**

**Office européen des brevets**

(11) Publication number: **0 382 531**
**A1**

(12) **EUROPEAN PATENT APPLICATION**

(21) Application number: 90301340.7

(22) Date of filing: 08.02.90

(51) Int. Cl.5: **A61K 39/012, C07K 15/04, C07K 3/00**

(30) Priority: **10.02.89 US 309644**

(43) Date of publication of application:
**16.08.90 Bulletin 90/33**

(84) Designated Contracting States:
**CH DE FR GB IT LI NL**

(71) Applicant: **MERCK & CO. INC.**
**126, East Lincoln Avenue P.O. Box 2000**
**Rahway New Jersey 07065-0900(US)**

(72) Inventor: **Gurnett, Anne M.**
**104 Wellington Place**
**Aberdeen, New Jersey 07747(US)**
Inventor: **Turner, Mervyn J.**
**918 Boulevard**
**Westfield, New Jersey 07090(US)**
Inventor: **St John Crane, Mark.**
**137 St Paul St.**
**Westfield, New Jersey 07090(US)**

(74) Representative: **Hesketh, Alan, Dr. et al.**
**European Patent Department Merck & Co.,**
**Inc. Terlings Park Eastwick Road**
**Harlow Essex, CM20 2QR(GB)**

(54) **Eimeria membrane associated protein immunogens.**

(57) Novel Eimeria membrane associated proteins with immunogenic properties are identified, purified and characterized. The cellular location of the membrane associated proteins is characterized by their partitioning properties in the detergent Triton X114 and by the presence on a subset of the proteins of a hydrophobic glycolipid anchor. Antiserum raised against detergent phase proteins reacts specifically with the surface of Eimeria sporozoites. The Eimeria proteins are capable of protecting poultry from coccidiosis.

EP 0 382 531 A1

## EIMERIA MEMBRANE ASSOCIATED PROTEIN IMMUNOGENS

BACKGROUND OF THE INVENTION

Coccidiosis is a disease caused by infection with one or more of the many species of coccidia, a subdivision of the phylum Protozoa. The coccidia are intracellular parasites which can infect a wide range of hosts and may result in severe economic loss to the sheep, goat, cattle, swine and poultry industry. Indeed, coccidiosis resulting from infection with Eimeria species has caused economically devasting losses to the poultry industry. Poultry is defined herein as domesticated birds that serve as a source of eggs or meat and that include among commercially important kinds chickens, turkeys, ducks, geese, guinea fowl, pheasants, pigeons and peafowl. Among domesticated birds, chicken production is the most susceptible to the economic losses from coccidiosis, although losses can also occur with turkeys, geese, ducks, and guinea fowl. Coccidiosis also produces serious losses in pheasants and quail raised in captivity. Coccidiosis may be acute and characterized by devastating flock mortality or the disease may be chronic and characterized by lack of weight gain.

Poultry are infected by coccidia following ingestion of the vegetative stage of the parasite, the sporulated oocyst. The infective stage, the sporozoite, is released in the intestine where it rapidly invades epithelial cells subsequently undergoing several generations of rapid intracellular asexual multiplication (schizogony) before entering the stage of sexual differentiation and mating (gametogony) leading to the formation of immature oocysts which are shed in the droppings and then undergo an extracellular sporulation process (sporogony) resulting in the generation of mature oocysts. Low level infection with any of the Eimeria species (spp.), E. acervulina, E. mivati, E. mitis. E. praecox. E. hagani. E. necatrix. E. maxima. E. brunetti and E. tenella results in a protective immunity to reinfection. There may be as many as twelve distinct cell types involved in the development of the parasite, each morphologically and antigenically different. At least three of these cell types have been shown to induce a protective immune response in the host, Rose and Hesketh, Parasitol. 73:25-37 (1976), McDonald et al., Parasitol. 93:1-7 (1986), Bhanushali and Long, In, Research in Avian Coccidiosis, Proc. of the Georgia Coccidiosis Conf., Athens, GA, USA pp. 526-534 (1986). Both the sporozoite as well as the first and second generation schizont appear to contain antigens which elicit an immunizing effect in chickens.

Unlike the sporozoite surface of other parasites such as Plasmodium falciparum which is composed of a single dominant antigen, Santoro et al., J. Biol. Chem. 158:3341-3345 (1983), the Eimeria spp., in particular, E. tenella, sporozoite surface appears to be antigenically complex, Wisher, Mol. Biochem. Parasitol. 21:7-15 (1986). Because the sporozoite stage cannot be cultivated in vitro and large amounts of sporozoite material would be necessary for conventional biochemical analysis and for subunit vaccine evaluation, the purification of these antigens has posed a problem. A subunit vaccine as used herein is defined as a peptide, polypeptide or protein which is either isolated from one or more of the life stages of any species of Eimeria or is produced by recombinant DNA technology and which either individually or combined with other such peptides, polypeptides or proteins induces a protective immunity in poultry following vaccination. The recombinant antigens or immunogens will be the same as or similar to the peptides, polypeptides or proteins isolated from one or more life stages of Eimeria. Immunogen is defined as a substance that when introduced into the body stimulates an immune response which is protective in nature, such AS the use of a vaccine to produce immunity against a microorganism. Immunity is defined as the non-susceptibility to the invasive or pathogenic effects of foreign organisms or the toxic effects of products of foreign organisms. The protective immunity may be either humoral or cell-mediated immunity. Humoral immunity is defined as specific immunity mediated by antibodies which are present in the plasma, lymph and tissue fluids of the body, and which may become attached to cells. Cell-mediated immunity is defined as specific immunity mediated by T lymphocytes. Antigen is used herein to define a substance capable of specifically combining with specific antibody.

Solubilized E. tenella sporozoite proteins, identified by monoclonal antibodies prepared against intact E. tenella sporozoites, have been shown to protect chickens against challenge with infective oocysts, Schenkel et al., European Patent Application Number 135,712. Similar results were obtained with E. tenella merozoites prepared by the same techniques, Schenkel et al. European Patent Application Number 135,073. Immunogenic polypeptides have been isolated from E. tenella sporozoites, Murray and Galuska, U.S. Patent Number 4,639,372. There was no indication, however, that any individual polypeptide would protect chickens against E. tenella challenge.

Recombinant DNA technology has allowed for the identification of immunogenic Eimeria polypeptides and for the production of the polypeptides in sufficient quantities for vaccine development. Newman et al.,

European Patent Application 164,176, describe the isolation of a 25,000 dalton polypeptide from E. tenella which is made up of two subunits of 17,000 and 8,000 daltons respectively. The 25,000 dalton polypeptide has been produced by recombinant DNA technology utilizing a genomic DNA clone and has been shown to protect chickens against coccidiosis caused by E. tenella. Another immunogenic E. tenella polypeptide has been disclosed by Anderson and McCandliss, Patent Cooperation Treaty Application WO 86/00528. This peptide has been sequenced and is composed of 280 amino acids, has been produced by recombinant DNA technology utilizing both an oocyst genomic DNA clone and a clone isolated from total oocyst mRNA, and protects chickens against coccidiosis. Clark et al., Mol. and Biochem. Parasit. 22:79-87 (1987), recently disclosed the construction of genomic DNA expression libraries from E. tenella in Escherichia coli using the expression vector λamp3. Clones expressing E. tenella immunogens were detected but none of the peptides were tested for immunogenic activity. Eimeria tenella sporozoite surface membranes have been labeled by various techniques to characterize potential surface immunogens, Wisher, Mol. Biochem. Parasit, 21:7-15 (1986). The major surface polypeptides which reacted with anti-E. tenella antibody were in the following molecular weight ranges: 113-96 kD, 73-67 kD, 54-42 kD, 37-32 kD, and 18-14 kD.

SUMMARY OF THE INVENTION

Novel Eimeria membrane associated proteins with immunogenic properties are identified, purified and characterized. The cellular location of the membrane associated proteins is characterized by their partitioning properties in the detergent Triton X114 and by the presence on a subset of the proteins of a hydrophobic glycolipid anchor. Antiserum raised against detergent phase proteins reacts specifically with the surface of Eimeria sporozoites. The Eimeria proteins are capable of protecting poultry from coccidiosis.

OBJECTS OF THE INVENTION

It is accordingly, an object of the present invention to provide novel membrane associated proteins of Eimeria species which can be used to immunize chickens against coccidiosis. Another object is to provide immunogenic proteins specifically associated with the membranes of unsporulated or sporulated oocysts and sporozoites. A further object is to provide a means for obtaining these membrane associated proteins. Another object is to provide a coccidiosis vaccine which is protective against those species of Eimeria mainly responsible for coccidiosis in poultry. A further object is to provide compositions for the prophylatic administration of the protein immunogens. These and other objects of the present invention will be apparent from the following description.

DETAILED DESCRIPTION OF THE INVENTION

The present invention relates to coccidiosis vaccines based on membrane associated native or recombinant-derived purified protein immunogens and any microheterogeneous or subunit·immunogen forms of the proteins associated with unsporulated or sporulated Eimeria oocysts and sporozoites. Native protein as used herein refers to the full length protein produced by the appropriate Eimeria gene in the parasite. Native protein is also intended to include the protein and any attached carbohydrate which was originally associated with the glycolipid membrane attachment anchor, Low, Biochem. J. 244: 1-13 (1987). Recombinant-derived refers to the isolation of a gene for a desired protein and the use of that purified gene to construct a bacterium or other cell type which will overproduce the desired protein. Subunit immunogen forms is defined as a portion of an immunogenic protein, polypeptide or glycoprotein which has fewer amino acids than the native immunogenic moiety but contains the immunogenic site or sites of the immunogen. Microheterogeneous forms as used herein refers to a single gene product, that is a protein produced from a single gene unit of DNA, which is structurally modified following translation. These structural modifications, however, do not result in any significant alterations of the immunogenic activity of the protein. The modifications may take place either in vivo, in the parasite, or during the isolation and purification process. In vivo modification may result in, but is not limited to, acetylation at the N-terminus, proteolysis, glycosylation or phosphorylation. Proteolysis may include exoproteolysis wherein one or more terminal amino acids are sequentially, enzymatically cleaved to produce microheterogeneous forms which have fewer amino acids than the original gene product. Proteolysis may also include endoproteolytic modification that results from the action of endoproteases which cleave the peptide at specific locations within the amino acid sequence. Similar modifications can occur during the purification process which may result in the production of microheterogeneous forms. The most common modification occuring during purification is proteolysis which is generally held to a minimum by the use of protease inhibitors.

The invention further relates to isolation and purification of the genetic information responsible for individual proteins and the methods of expressing the corresponding immunogenic proteins. Polypeptide or protein as used herein refers to a linear polymer of amino acids bound together with amide linkages. The sequence of amino acids in the chain is of critical importance in the biological functioning of the protein or polypeptide. Polypeptide and protein are used interchangeably herein. Immunogen as used herein refers to molecules or macromolecules which, when introduced into an animal body, stimulates a humoral and/or a cellular immune response which is functional in nature, that is an immunity which protects the animal from a specific infection. In the instant case an immunogen will produce an immune response, either humoral, cellular or both which will protect poultry against infection with Eimeria species which cause coccidiosis.

Eimeria oocysts are isolated from either the feces or the cecal contents of chickens orally infected 4 to 10 days earlier, depending on the species, with about $7.5 \times 10^4$ sporulated oocysts. The cecal contents or feces are physically disrupted in a Waring Blender, in distilled water and digested with a proteolytic enzyme, preferably pepsin, about 20 mgs/ml, pH 2.0. Debris and pepsin are removed by centrifugation in distilled water. A partially pure oocyst fraction is collected by flotation in about 1.1 M sucrose, Jackson, Parasitol. 54:87-93 (1964), and further treated by incubation in sodium hypochlorite at a concentration of about 5 to about 6 percent, preferably 5.25%, in water at about 4°C for approximately 10 minutes. The sodium hypochlorite is removed by several washes in sterile phosphate buffered saline (PBS) at about pH 7.6 to obtain purified, sterile oocysts. Oocysts about $2 \times 10^7$/ml in phosphate buffered saline (PBS) plus antibiotic/antimycotic (Gibco) are allowed to sporulate in a shaking water bath for about 36 hours at about 29°C, Edgar, Trans. Am. Micr. Soc. 62:237-242 (1954).

Sporozoites are prepared from sporulated oocysts using the technique of Patton and Brigman, J. Parasitol. 65: 526-530 (1979), with a modification by Dulski and Turner, Avian Diseases 32: 235-239 (1988). Sporulated oocysts are disrupted by shaking with glass beads, about 3mm, about $1 \times 10^8$ cells per ml in PBS. Sporocysts are purified by density gradient centrifugation in about 50% Percol for about 1 minute at about 10,000 x g to about 20 minutes at about 17,600 x g. The pelleted sporocysts, about $1 \times 10^7$ cells per ml, are resuspended in excysting solution, about 0.25% trypsin, about 4% taurodeoxycholic acid, and 10 mM MgCl$_2$ and incubated at about 41°C for about 60 minutes. The released sporozoites are collected by centrifugation at about 700 x g for about 10 minutes, washed once in PBS, the pellet is resuspended in about 50% Percol, about $1 \times 10^8$ cells per ml, and centrifuged again. The purified sporozoites are washed in PBS and collected by centrifugation.

Cloned cell lines of Trypanosoma brucei exhibiting antigenic types MITaR 1, described by Cross, Parasitol. 71:393-417 (1975), and ILTaR 1 serodemes, described by Miller and Turner, Parasitol. 82:63-80 (1981), are used for the isolation of variant surface glycoproteins (VSGs) and for the isolation of a phosphatidylinositol specific phospholipase C like activity (PIPLC, T. brucei lipase), Hereld et al., J. Biol. Chem. 261 : 13813-13819 (1986). Trypanosomes are purified from the blood of infected rats according to the method of Lanham and Godfrey, Exp. Parasitol. 28:521-534 (1970). Standard preparations of soluble VSG (sVSG) and membrane form VSG (mfVSG) are prepared by the methods of Cross, J. Cell Biochem. 24:79-90 (1984) and Gurnett et al., Mol. and Biochem. Parasitol. 20:1-13 (1986), respectively. A semi-purified preparation of T. brucei containing an active trypanosome lipase is prepared from MITat 1.6 trypanosomes or any suitable T. brucei clone. The trypanosomes are lysed in about 0.3 mM CaCl$_2$, about $1 \times 10^9$ cells per ml, at about 0°C for about 5 minutes. The lysate is centrifuged at about 3,000 x g for about 5 minutes, the supernatant fluid is removed and the pellet is resuspended at the equivalent of about $1 \times 10^9$ cells per ml in about 10 mM sodium phosphate, pH about 8.0, containing about 0.1 mM Nα-p-tosyl-1-lysine chloromethylketone (TLCK), and 0.1 mM dithiothreitol (DTT). The mixture is incubated at about 37°C for about 15 minutes, after which it is rapidly cooled to about 0°C and centrifuged at about 10,000 x g for about 15 minutes. The pellet is washed twice in 50 mM N-2-Hydroxyethylpiperazine-N'-2-ethanesulfonic acid (Hepes), 1 mM DDT, 0.1 mM TLCK, about $2.5 \times 10^9$ cell equivalents/ml, and is centrifuged at about 100,000 x g for about 1 hour. The pellet is resuspended in about the same volume, about $3.3 \times 10^9$ cell equivalents/ml of 50 mM Hepes, about pH 7.4, about 1% N-octyl-β-D-glucopyranoside (nOG), and centrifuged again at about 100,000 x g for about 1 hour. The final supernatant fluid contains an active lipase which is diluted about 1:10 in about 50 mM Hepes, pH about 7.4 and about 1% nOG prior to use. This fraction is termed T. brucei lipase.

Eimeria lysates are prepared by suspending unsporulated oocysts or sporulated oocysts, about $5 \times 10^7$ per ml in water and mixing with glass beads, about 3 mm diameter, for about 5 minutes at room temperature using a vortex mixer. The suspension is checked for complete breakage of oocysts and

4

EP 0 382 531 A1

sporocyst walls by light microscopy and, if necessary, mixed for a further 5 minute interval to ensure complete lysis. Sporozoites about $4 \times 10^8$ per ml in water are lysed by sonication.

Anti-cross reacting determinant (CRD) antibody is raised by injecting rabbits with purified ILTat 1.25 sVSG, about 1mg in PBS, about 250μl, is homogenized with an acceptable adjuvant and injected subcutaneously into multiple sites on the back of one or more rabbits. A second injection of about 1mg sVSG in about 250μl of PBS is given about 2 weeks later. Two weeks after the final injection blood is collected and serum isolated by standard procedures. The serum is collected and diluted about 1:1 in 0.1 M borate, about pH 8.0 and passed over a heterologous sVSG column of MITat 1.6 sVSG bound to Reacti Gel 6X (Pierce Chemicals). The flow through is discarded and the anti-CRD antibodies are eluted using about 0.1 mM Glycine-HCl, about pH 2.4. The antibodies are used at about 1:50 dilution, about 4 μg per ml for Western blotting.

Eimeria unsporulated oocyst, sporulated oocyst and sporozoite lysates, about 20 μl are treated with the T. brucei lipase. The lysates are mixed with about 2μl of buffer consisting of about 500 mM Hepes, pH 7.4, containing about 10% nOG, 1 mM phenylmethanesulfonyl fluoride (PMSF) and 10 mM TLCK. Lipase, about $1 \times 10^6$ cell equivalents, is added to lysate and incubated for about 20 min at about 37°C. Following incubation Laemmli sample buffer, see below, is added to each sample and they are boiled for three minutes prior to sodium dodecyl sulfate (SDS) polyacrylamide gel electrophoresis (PAGE) on gradient gels, 7-20% acrylamide, according to the method of Laemmli, Nature 227:630-684 (1970). After electrophoresis the proteins are characterized by Coomassie Brilliant Blue R staining or transferred to nitrocellulose for subsequent detection using antibodies, the latter known in the art as Western blotting according to the procedure of Towbin et al., Proc. Natl. Acad. Sci. USA:430-435 (1979). The transferred lysates are reacted with anti-CRD antibody which reacts specifically with the carbohydrate portion of the VSG glycolipid anchor. Bound antibody is detected by the addition of [$^{125}$I] protein A (Amersham) about $1 \times 10^6$ counts per minute per ml, 30 mCi per mg protein, followed by autoradiography. All lysates contain proteins capable of binding anti-CRD antibody, but binding occured only after treatment of the lysates with the T. brucei lipase.

To test the hydrophobicity of Eimeria anti-CRD binding proteins, lysates are subjected to phase separation in Triton X114 according to the method of Bordier, J. Biol. Chem. 256:1604-1607 (1981). This process takes advantage of the cloud point of Triton X114 to form a detergent-rich fraction containing the majority of the hydrophobic protein, and a detergent-depleted fraction containing the majority of the hydrophilic protein. Lysates of unsporulated or sporulated oocysts, about $2.5 \times 10^6$ cells per ml, and sporozoites, about $2 \times 10^7$ cells per ml, are incubated at about 0°C for about 1 hour in about 1% precondensed Triton X114, about 10 mM Tris, pH about 7.4, about 150 mM NaCl and about 1 mM PMSF. Following incubation, the samples are centrifuged for about 1 hour at about 100,000 x g and the supernatant fluid collected. The Triton X114 supernatants are either carefully layered over an equal volume of about 6% sucrose containing about 10 mM Tris, pH 7.4, and 150 mM NaCl or incubated without further treatment. Incubation at about 30°C for about 15 minutes results in a cloudy solution from which phases are separated by centrifugation at about 1,000 x g for about 15 minutes. Three fractions are collected; the top aqueous phase, middle sucrose portion, and the bottom detergent phase. Where no sucrose is used there is no middle phase. Each fraction is precipitated with about 4 volumes of ice cold acetone and prepared for analyis by SDS-PAGE and Western blot.

Antiserum is produced against the contents of the detergent phase of the Eimeria sporozoites. Blood is collected from the antibody producing animals, preferably rabbits, prior to initiation of the immunization procedure and the preimmune serum is isolated and stored for control purposes. The rabbits are given multiple immunization injections with the detergent phase protein immunogens, about 20 μg to about 80 μg of protein per immunization, with about 50μg protein preferred. The initial immunization is given with an acceptable adjuvant, generally equal volumes of immunogen and adjuvant. Acceptable adjuvants include Freund's complete, Freund's incomplete, alum-precipitate, water-in-oil emulsion containing Corynebacterium parvum and tRNA, with Freund's complete adjuvant being preferred for the initial immunization. Freund's incomplete adjuvant is preferred for all booster immunizations. The initial immunization consists of the administration of about 1 ml of emulsion at multiple subcutaneous sites on the backs of the rabbits. Booster immunizations utilizing an equal volume of immunogen are given at about one month intervals and are continued until adequate levels of antibodies are present in an individual rabbit's serum, generally on about days 30 and 90. Blood is collected and serum isolated by methods known in the art. The antisera raised against coccidial proteins found in the TX114 detergent phase is designated anti-TX114B. The rabbit anti-TX114B antiserum is characterized by Western blot analysis using antigens from unsporulated and sporulated oocysts and sporozoites and is used for indirect immunofluorescence using sporozoites and by an agglutination assay using sporozoites. Antigen as used herein is defined as any substance that can combine with an antibody. Immunogens as described above are considered antigens when used to

5

EP 0 382 531 A1

characterize the specific antibody.

Acetone precipitated phase separated lysates of Eimeria unsporulated or sporulated oocysts are resuspended in Hepes, 50mM, pH 7.4, 1% nOG (about $1 \times 10^6$ cell equivalents per about 20 $\mu$l), aqueous phase, cushion and detergent phase are treated with T. brucei lipase (about 5$\mu$l) and analysed by SDS-PAGE and Western blot analysis. Sporozoite samples are prepared in the same way except about $8 \times 10^6$ cell equivalents per 20$\mu$l are used. The majority of the anti-CRD binding proteins from all three stages are concentrated in the bottom detergent phase.

Treatment of the lysates with the trypanosome lipase should change the hydrophobic CRD-bearing proteins into water soluble molecules. Lysates are subjected to Triton X114 treatment either before or after lipase treatment. Western blot anlaysis with anti-CRD antibody reveals that when the lipase is added before phase separation, the CRD-bearing proteins are found in the top aqueous phase and if the lipase is added after phase separation the same proteins are found in the detergent phase. When anti TX114B antiserum is substituted for anti-CRD antibody, most of the proteins recognized by anti-TX114B are shifted to the aqueous phase when lipase treatment precedes phase separation. Since these proteins co-migrate with the proteins recognized by anti-CRD antibodies, it can be concluded that most of the proteins recognized by anti-TX114B are glycolipid bearing proteins.

Molecular weights and isoelectric points of Eimeria proteins reactive with both anti-CRD antibody and anti-TX114B antibody are determined. Molecular weights are determined by analytical SDS-PAGE followed by transfer to nitrocellulose and immunodetection by Western blotting with the appropriate antibody. Appropriate molecular weight controls are included. Isoelectric points are determined by Western blotting of two dimensional gels run according to the procedure of 0'Farrell, J. Biol. Chem. 250:4007-4021 (1975), with modifications similar to the method of Groppi, Mol. Pharmacol. 18: 429-437 (1980). Antibodies for both procedures are prepared as stated above. The results are shown in Table 2.

An indirect immunofluorescence assay is carried out by reacting heat inactivated anti-TX114B antiserum, diluted about 1:50, final volume about 200 $\mu$l, with viable purified sporozoites, about $5 \times 10^6$, for about 1 hour at about 4° C in about 0.05% (w/v) bovine serum albumin (BSA) in PBS. The sporozoites are washed three times with 0.05% BSA in PBS and pelleted by centrifugation at about 1,000 x g, for about 10 minutes, at about 4° C between each wash. The washed sporozoites are fixed with about 2.5% formaldehyde for about 5 minutes, washed twice with about 0.05% BSA in PBS and stained for about 30 minutes at about 4° C with diluted fluorescein conjugated goat anti-rabbit IgG (Miles Laboratories), diluted about 1:15. After 3 washes with about 0.05% BSA in PBS, the sporozoites are collected by centrifugation and resuspended in about 25 $\mu$l of about 66% (v/v) glycerol in about 50 mM Tris-HCl, about pH 9.0 prior to viewing by either phase or fluorescence microscopy, techniques known in the art. Anti-TX114B antibody treated sporozoites show a diffuse stainng pattern on the surface of the cells while preimmune serum reveals no background immunofluorescence.

Since anti-TX114B antiserum appears to agglutinate Eimeria sporozoites when antibody activity is determined by immunofluorescence the antiserum is tested for agglutination activity. The agglutination assay is performed using Linbro 96-round-bottom-well microtiter plates. Mixtures, about 0.1 ml, of heat inactivated antisera diluted in Eagle's minimal essential medium (MEM) and purified sporozoites, about $2 \times 10^5$ cells per well, are seeded in duplicate and incubated for about 1 hour at about 4° C. Following incubation, about 0.01 ml of glutaraldehyde in MEM, to give a final concentration of about 0.5% glutaraldehyde, is added to each well and the contents of each well is counted using a Coulter counter, model ZBI. The agglutination titration curve of preimmune and TX114B sera is shown in Figure 1. The immue sera strongly agglutinates purified sporozoites.

A detergent rich fraction as prepared above is used to isolate and purify glycolipid linked proteins. An aliquot of about 5 mg of protein is precipitated with a ketone such as acetone, about 4 volumes of cold acetone to about 1 volume of the detergent rich fraction, precipitated overnight at about -20° C, pelleted by centrifugation at about 10,000 x g for about 15 minutes. The pelleted material is resuspended in Buffer A which consists of bis[2-hydroxyethyl]imino-tris-[hydroxymethyl]-methane, about 4 ml, Bis Tris pH 6.9, containing 0.05% TX100. The insoluble material is removed by centrifugation, about 13,000 x g for about 2 min, and the supernatant fluid subjected to anion exchange high performance liquid chromatography (HPLC). The proteins were applied to a BioGel TSK, DEAE-5PW column (Biorad) and eluted with about 100 mM Bis Tris pH 5.8 containing about 0.05% TX100. Aliquots of the fractions collected are analyzed by polyacrylamide gel electrophoresis using the procedure of Laemmli and either silver stained to confirm the number and molecular weight of the proteins or transferred to nitrocellulose and probed with anti-CRD antisera to identify the glycolipid bearing proteins. The number and molecular weights of the major glycolipid linked proteins is confirmed. Fractions containing glycolipid linked proteins are used to prepare material for protein sequencing or for the production of specific antibodies.

6

The material transferred to the nitrocellulose is treated with T. brucei lipase prior to antibody treatment. The nitrocellulose is washed with a Tris-HCl buffer, about 50 mM, about pH 7.4, containing EDTA, about 5 mM, and sodium chloride, about 150 mM. The cellulose is rinsed with Hepes, about 20 mM, pH about 7.3 and excess buffer removed by blotting. Lipase (T. brucei lipase as described previously) is diluted 1:10 with Hepes/nOG, about 20 mM Hepes, pH 7.3, about 1% n-octyl glucoside, total volume of about 3 ml, and sealed in a bubble free plastic bag with the nitrocellulose. This is incubated for about 2 hours at about 37°C with occasional agitation. The lipase is discarded and the nitrocellulose is probed with anti-CRD antibody. The glycolipid nature of a subset of the proteins in the column fractions is confirmed.

One of the four major glycolipid linked proteins, 26kD, is obtained in a pure form in the column fractions, as demonstrated by silver strain gels. This protein is used directly in amino terminal sequence analyses (Table 3) and is able to protect chickens against a coccidiosis infection (Table 4).

Purified glycolipid linked proteins separated by polyacrylamide gel electrophoresis are used to produce protein specific antibody. Following electrophoresis, the gels are stained with Commassie Brilliant Blue R, about 0.1% in 20% methanol and 7% acetic acid, and destained with about 20% methanol and 7% acetic acid. Bands of interest are excised, dried by lyophilization and macerated in about 0.5 ml of phosphate buffered saline PBS. About 3 to about 15 $\mu$g of protein is used in each 0.5 ml aliquot. The purified proteins are mixed with adjuvant and injected subcutaneously into animals as described above. The animals are boosted and the antisera collected as previously described.

Amino terminal amino acid sequencing of the purified glycolipid linked proteins is accomplished essentially by the process of Matsudaira, J. Biol. Chem. 262: 10035-10038 (1987). Samples of the 32 kD and 29 kD proteins are separated on 7-20% polyacrylamide gels according to the procedure of Laemmli. The gels are soaked in transfer buffer (about 10 mM 3-[cyclohexylamino]-1-propanesulfonic acid, Caps, about 10% methanol, about 5 mM DTT) for about 5 minutes and electrophoretically transferred to polyvinyldifluoride membranes (PVDF)(Immobilon, 0.45 $\mu$M pore, Millipore) at about 0.5 Amperes for about 1.5 hours. The PVDF membranes are washed, stained with Commassie Brilliant Blue R, destained, washed and air dried. The bands of interest are excised and sequenced.

Poultry are administered an immunizing dosage of one or more of the native or recombinant derived Eimeria membrane associated immunogens described above. Immunogen administration to chickens may be by oral or parenteral routes or chicken embryos may be inoculated through the egg shell. Administration of immunogen by any of these routes may include an immunogen or immunogens given alone or as a solution or suspension with a physiologically acceptable medium. Such physiologically acceptable media include, but are not limited to, physiological saline, phosphate buffered saline, phosphate buffered saline glucose, buffered saline and the like. Parenteral administration includes inter alia, intramuscular, intraperitoneal, subcutaneous and intravenous injection for delivery of the Eimeria membrane associated immunogens. Orally administered immunogens can be in the form of an aqueous solution or suspension. A suspension may include the immunogen in a gel composed of, for example, gelatins or alginates. Orally administered immunogens may also be included in the feed. Embryonated eggs are immunized by the injection of an immunogenic dose of one or more of the Eimeria immunogens. The immunogens for intramuscular and subcutaneous vaccination may be given along with an acceptable adjuvant. Acceptable adjuvants include, but are not limited to, Freund's complete, Freund's incomplete, double emulsions, anhydrous oils, alum-precipitate, water-in-oil emulsion containing Corynebacterium parvum and t-RNA. The preferred adjuvant is alum-precipitate, in which the immunogen has been precipitated with aluminum hydroxide such as Alhydrogel™. Immunization of chickens with Eimeria membrane associated protein immunogens results in immunity to coccidiosis. Protective immunity is achieved by administration of from about 1.0 ng to about 100 $\mu$g, with about 100 ng to about 10 $\mu$g being preferred.

The following examples illustrate the present invention without, however, limiting the same thereto.

<u>EXAMPLE 1</u>

Preparation of Oocysts Sporulated Oocysts and Sporozoites

Chickens were infected orally with 7.5 x 10$^4$ E. tenella sporulated oocysts. The parasites were harvested from the ceca seven days post infection. Unsporulated oocysts were prepared using the method of Jackson (Jackson, 1964). Briefly, the cecal contents were homogenized and treated with pepsin (20 mgs/ml, pH 2.0) at 37°C. The unsporulated oocysts were pelleted (1700 x g for 10 minutes) and then resuspended in 1 M sucrose. Following centrifugation (1700 x g for 10 minutes), the floating oocyst layer was resuspended in water and pelleted (1700 x g for 10 minutes). The pellet was washed in water and resuspended in cold 5.25% sodium hypochlorite. After 10 minutes on ice, the sodium hypochlorite was removed using 5 washes in water. The unsporulated oocyst pellet was finally resuspended in PBS. The cells [2 x 10$^7$/ml in PBS plus antibiotic/antimycotic (Gibco)] were sporulated by incubation at 29°C with continual agitation over a 36 hour period. Sporozoites were prepared from the sporulated oocysts, Patton and Brigman, J. Parasitol. 65: 526-530 (1979), using a modification by Dulski and Turner, Avian Diseases 32: 235-239 (1988). Sporulated oocysts were shaken with glass beads (3 mm diameter beads, 1 x 10$^8$ cells/ml in PBS). Sporocysts were purified by density gradient centrifugation in 50% Percol (1 minute, 10,000 x g in the microfuge or 17,600 x g for 20 minutes, dependent on the size of the preparation). The pelleted sporocysts were resuspended in excysting solution (1 x 10$^7$ cells/ml, 0.25% trypsin, Gibco, 4% taurodeoxycholic acid, 10 mM MgCl$_2$) and incubated at 41°C for 60 minutes. The released sporozoites were pelleted (700 x g, 10 minutes), washed once in PBS, resuspended in 50% Percoll (1 x 10$^8$ cells/ml), and centrifuged through Percoll as above. Finally, the pelleted sporozoites were washed once in PBS.

## EXAMPLE 2

### Trypanosomes. VSG Purification and Lipase Preparation

Trypanosomes used were cloned antigenic types of the MITaR 1, Cross, Parasitol. 71: 393-417 (1975), and ILTaR 1 serodemes, Miller and Turner, Parasitol. 82: 63-80 (1981). Trypanosomes were purified from the blood of infected rats according to the method of Lanham and Godfrey, Exp. Parasitol. 28: 521-534 (1970). Standard preparations of sVSG and mfVSG were prepared by the methods of Cross, J. Cell Chem. 24: 79-90 (1984) and Gurnett et al. Mol. biochem. Parasitol. 20: 1-13 (1986) respectively. A semi-purified fraction containing an active trypanosome lipase was prepared from MITat 1.6 trypanosomes (or any suitable T. brucei clone). Trypanosomes were lysed in 0.3 mM CaCl$_2$ at 1 x 10$^9$ cells/ml, and kept at 0°C for 5 minutes. The lysate was centrifuged at 3,000 x g for 5 minutes and the pellet resuspended at 1 x 10$^9$/ml in 10 mM sodium phosphate, pH 8.0, containing 0.1 mM TLCK, 0.1 mM DTT. After 15 minutes incubation at 37°C, the mixture was cooled rapidly to 0°C and centrifuged at 10,000xg for 15 minutes. The pellet was washed twice in 50 mM Hepes, 1 mM DTT, 0.1 mM TLCK (2.5 x 10$^9$ cell equivalents/ml) and was then centrifuged at 100,000xg for 1 hour. The pellet was resuspended in 50 mM Hepes, pH 7.4, 1% nOG, (3.3 x 10$^9$ cell equivalents/ml) and spun again at 100,000xg for 1 hour. The final supernatant contained an active lipase which was diluted 1:10 in 50 mM Sepes, pH 7.4, 1% nOG prior to use in these experiments.

## EXAMPLE 3

### Production of Anti-cross Reacting Determinant (CRD) Antibody

Purified ILTat 1.25 sVSG from, Example 2, was used to raise antibodies in rabbits. One mg sVSG in PBS (250µl) was homogenized with Freunds complete adjuvant and injected subcutaneously into multiple sites on the back of a rabbit. A second injection of sVSG (1mg) in 250µl of PBS was given 2 weeks later. Blood was taken and serum isolated after a further 2 weeks using standard techniques. The serum was diluted 1:1 in 0.1 M borate, pH 8.0 and passed over a heterologous sVSG column of MITat 1.6 sVSG bound to Reacti Gel 6X (Pierce Chemicals). The flow-through was discarded and the anti-CRD antibodies were eluted using 0.1 mM Glycine-HCl, pH 2.4. The antibodies were used at 1:50 dilution (about 4 ug/ml) for Western blotting. Western blotting was carried out on sodium dodecyl sulfate polyacrylamide gel (SDS-PAGE) separated proteins. SDS polyacrylamide gels, 7-20%, were prepared according to the method of Laemmli, Nature 227: 630-684 (1970). After electrophoresis, gels were either stained using Coomassie Brilliant Blue R or transferred to nitrocellulose for subsequent detection using antibodies, Towbin et al., Proc. Nat'l. Acad. Sci. USA 76: 4350-4354 (1979). Proteins were transferred in a water cooled Hoeffer apparatus (Hoeffer Scientific Instruments, San Francisco, California) for 16 hours at 0.2 A. Bound antibody

8

was detected using $^{125}$I protein A (Amersham, 1 x 10$^6$ cpm/ml, 30 mCi/mg protein), followed by autoradiography.

EXAMPLE 4

E. tenella Proteins Contain Determinants In Common With the CRD of T. brucei VSG

Lysates of unsporulated oocysts, sporulated oocysts and sporozoites of E. tenella, from Example 1, treated with T. brucei lipase were subjected to SDS-PAGE and Western blotting using the rabbit anti-CRD antibody, from Example 3, directed against the carbohydrate portion of the VSG glycolipid anchor. A subset of proteins from each stage bound to the antibody showing that there are a number of proteins in E. tenella which possess determinants in common with the CRD of T. Brucei VSG. The fact that the glycoproteins do not bind to the antibody before lipase treatment is in agreement with VSG studies (Cardoso de Almeida and Turner, Nature 302: 349-352 (1983). No difference was observed in the Coomassie Blue staining pattern when comparing E. tenella lysates treated with or without the trypanosome lipase.

EXAMPLE 5

Phase Separation of E. tenella lysates

Unsporulated oocysts or sporulated oocysts (from Example 1), 5 x 10$^7$/ml in water, were vortex mixed with glass beads (3 mm diameter) for five minutes at room temperature. The lysates were checked for complete breakage of oocyst and sporocyst walls under the microscope and, if necessary, vortex mixed for a further five minutes to ensure complete lysis. Lysates were frozen at -70°C for further use. Sporozoites (4 x 10$^8$/ml in water) were lysed by sonication (Branson Sonifier, set at 20 power units, 20 secs. using 50% "on" time), and frozen at -70°C until further use.

Proteins of E. tenella were subjected to phase separation in Triton X114 according to the method of Bordier, J. Biol. Chem. 256: 1604-1607 (1981). Lysates of unsporulated oocysts, sporulated oocysts (2.5 x 10$^6$ cells/ml) and of sporozoites (2 x 10$^7$ cells/ml) were incubated on ice for 1 hour in 1% precondensed Triton X114, 10 mM Tris, pH 7.4, 150 mM NaCl and 1 mM PMSF. After incubation, the samples were centrifuged for 1 hour at 100,000xg and the pellets discarded. The supernatant was carefully layered over an equal volume of a 6% sucrose cushion containing 10 mM Tris, pH 7.4, 150 mM NaCl, or incubated without further treatment. Incubation at 30°C for 15 minutes caused the formation of detergent micelles and the phases were separated by centrifugation at 1000xg for 15 minutes. Three fractions were collected; the top aqueous phase, middle sucrose cushion, and bottom detergent phase. Where no sucrose was used there was no middle phase. Each fraction was acetone precipitated using 4 volumes of ice cold acetone, in preparation for gel electrophoresis.

Hydrophobic proteins are concentrated in the detergent-rich phase, whereas hydrophilic ones tend to be found in the upper aqueous phase. The majority of the anti-CRD binding proteins from all 3 stages tested appear to be concentrated in the bottom detergent phase as might be expected. In the absence of a suitable lipase, all the anti-CRD binding proteins should be hydrophobic.

Glycoproteins bearing epitopes in common with the glycolipid anchor of VSG are concentrated in the detergent-rich phase, but do not constitute all the proteins found in this fraction. In the sporulated oocyst and sporozoite, the detergent soluble anti-CRD binding proteins appear to make up a high proportion of the bands seen on the Coomassie Blue stained gel. In the case of the unsporulated oocysts the anti-CRD binding proteins are a minor fraction of the total protein in the detergent rich phase. When the lipase is added before phase separation, the CRD bearing proteins are found in the top aqueous phase. Lipase treatment after phase separation leaves the same proteins in the detergent phase. Proteins that are associated with the detergent-rich TX114 fraction are hydrophobic and are likely to be membrane bound.

EXAMPLE 6

9

E. Tenella lysates treated with T. brucei lipase before and after phase separation

E. Tenella unsporulated oocysts, sporulated oocysts and sporozoites were lysed in water as previously described (unsporulated and sporulated oocysts 5X10⁷/ml, sporozoites 4 x 10⁸/ml). 100µl of 500mM Hepes, pH 7.4, 10% nOG, 10mm TLCK was added to 1ml of each lysate. An aliquot (80µ1) was removed from each lysate and mixed with 20µl of T. brucei lipase (set 1). A second aliquot (80µl) was removed from each lysate and mixed with 20µl of 50mM Hepes, 1% nOG as a no enzyme control (set 2). The aliquots were incubated at 30°C for 30 minutes, then mixed with 2 mls of 1% TX114 containing 10mm Tris pH 7.4, 150mM NaCl, 1mM PMSF. The samples were incubated on ice for 1 hour, centrifuged at 100,000Xg for 1 hour and the supernatant collected. Each supernatant was carefully layered onto an equal volume of 6% sucrose containing 10mM Tris pH 7.4, 150mM NaCl, incubated for 15 minutes at 30°C and centrifuged (1000xg for 15 minutes) to separate the phases. Three fractions were collected, top aqueous phase, middle sucrose phase and bottom detergent phase. Water was added to equalise the volumes, and proteins precipitated by the addition of four volumes of ice cold acetone, incubation overnight at -20°C, followed by centrifugation at 10,000xg for 15 minutes. The pellets from set 1 were resuspended in 80µl of 50mM Hepes pH 7.4, 1% nOG and treated with 20µl of the same buffer. Pellets from set 2 were resuspended in 50mM Hepes pH 7.4, 1% nOG and treated with 20µl of T. brucei lipase. After incubation at 30°C for 30 minutes an equal volume of Laemmli sample buffer was added to each sample in preparation for PAGE. 25% of each sample was loaded per track, proteins were transferred to nitrocellulose and probed with anti-CRD or with anti-TX114B.

The data with anti-CRD shows that when lysates are lipase treated after phase separation the hydrophobic glycolipid linked proteins are found in the detergent phase. These proteins are found in the aqueous phase when the lipase, which changes their hydrophobic nature, is added prior to phase separation.

Blots probed with anti-TX114B reveal a very similar pattern, suggesting that the majority of proteins in the TX114B are glycolipid linked (See also Example 7).

EXAMPLE 7

Production and Use of Anti-TX114B Antiserum

An antiserum was prepared against the contents of the detergent phase prepared from sporozoites, (See Example 5) by injection of 50 ug of protein in complete Freunds adjuvant subcutaneously into multiple sites on the back of a rabbit, with boosts of 50 ug in incomplete Fruends adjuvant on days 30 and 90. The antiserum is referred to as anti-TX114B.

Anti-TX114B anti serum was used to blot similar gels as prepared in Example 5. The results show that most of the proteins in sporulated oocysts and sporozoites recognized by anti-TX114B are shifted to the aqueous phase following lipase treatment prior to phase separation. Since these proteins co-migrate with those recognized by anti-CRD, it follows that most of the proteins recognized by anti-TX114B are glycolipid bearing.

Indirect immunofluorescence - Rabbit anti-TX114B antiserum was heat inactivated at 56°C for 30 minutes and was incubated at a dilution of 1:50 in a final volume of 200 ul with viable purified sporozoites (5 x 10⁶ sporozoites) for 1 hour at 4°C in 0.05% BSA in PBS. The sporozoites were washed three times with 0.05% BSA in PBS and pelleted by centrifugation (1000 x g, 4°C, 10 minutes) between each wash. The sporozoites were fixed with 2.5% formaldehyde for 5 minutes, washed twice with 0.05% BSA in PBS, and stained with diluted fluorescein conjugated goat anti-rabbit IgG (1:15, Miles Lab., Elkhart, Indiana) for 30 minutes at 4°C. After 3 washes with 0.05% BSA in PBS, the sporozoites were collected and resuspended in 25 ul of 66% (v/v) glycerol in 50 mM Tris-HCl, pH 9.0 prior to viewing using a Leitz microscope (Vario Orthomat). Immunofluorescence of sporozoites with heat inactivated rabbit anti-TX114B showed an overall, diffuse staining pattern on the surface of sporozoites after fixation with formaldehyde and detection with FITC-conjugated anti-rabbit IgG. The preimmune serum of the rabbit gave no background immunofluorescence. The rabbit anti-CRD gave no immunofluorescence or agglutination.

Agglutination - The titration curve of agglutination activity of rabbit anti-TX114B and rabbit prebleed serum are shown in Table 1. Agglutinated parasites pass through the Coulter aperture together and are counted as a single particle. Thus, agglutination is inversely proportional to the Coulter count. At very low titers, the preimmune serum slightly agglutinated the sporozoites. The rabbit anti-CRD gave no agglutination by the same assay.

Table 1

| AGGLUTINATION OF SPOROZOITES WITH ANTI-TX114B | | |
|---|---|---|
| Dilution of Serum | Pre-immune Serum | Anti-TX114B |
| 1:25 | .835 | .228 |
| 1:250 | .882 | .291 |
| 1:500 | .974 | .399 |
| 1:1000 | .987 | .536 |
| 1:2000 | .999 | .718 |
| 1:4000 | .997 | .833 |
| 1:8000 | 1.030 | .924 |
| 1:16,000 | .927 | .935 |
| 1:32,000 | .974 | .932 |
| 1:64,000 | .963 | .926 |
| 1:128,000 | .982 | .949 |
| a) The no serum control gave a reading of 7230 | | |
| b) Data presented as: Coulter counter reading divided by 7230 (no serum control) | | |
| c) Values are inversely proportional to agglutination | | |

EXAMPLE 8

Purification of Glycolipid Linked Proteins

A detergent rich fraction was prepared from E. tenella sporozoites (TX114B), Example 5. An aliquot of this fraction containing 5 mg of protein was precipitated with acetone (4 volumes of ice cold acetone, 1 volume of TX114B, precipitated overnight at -20°C, pelleted by centrifugation at 10,000 x g for 15 minutes, supernatant discarded). The resultant pellet was resuspended in bis[2-hydroxyethyl] imino-tris-[hydroxymethyl]methane (4 mls, 100 mM Bis Tris pH 6.9) containing 0.05% TX100 (Buffer A). Insoluble material was removed by centrifugation (13,000 x g for 2 minutes) and the supernatant applied to an anion exchange HPLC column (BioGel TSK, DEAE-5PW, 75 x 7.5 mm, Biorad). The HPLC was comprised of a Waters 600E solvent delivery system, Waters 481 detector, and a U6K injection port. Proteins were eluted from the column using 100 mM Bis Tris pH 5.8 containing 0.05% TX100; the column effluent was monitored at 280 nm, and 1 ml fractions were collected. Aliquots of these fractions were analyzed on polyacrylamide gel according to the method of Laemmli and either silver stained or transferred to nitrocellulose and probed with anti-CRD. In the latter case, the nitrocellulose was treated with a fraction containing lipase from T. brucei prior to probing with the antibody. The nitrocellulose was first washed with TEN (50 mM Tris-HCl, pH 7.4, 5 mM EDTA, 150 mM NaCl, 3 washes of 200 mls), then rinsed (100 mls) with Hepes (20mM, pH 7.3). Excess buffer was removed by blotting with filter paper. A fraction containing T. brucei lipase (Example 2) was diluted 1:10 with Hepes/nOG (50 mM Hepes, pH 7.3, 1% n-octyl glucoside, total volume 3 mls) and sealed in a bubble free plastic bag together with the nitrocellulose sheet. This was then incubated for 2 hours at 37° C with occasional agitation of the bag. The lipase was discarded and the nitrocellulose probed with anti-CRD as described in examples. Fractions containing glycolipid linked proteins were used to prepare material for protein sequencing or in the production of antibodies. The results are shown the following table. Silver stained gels showed that some fractions from the anion exchange column contained pure 26kD glycolipid linked proteins. Several fractions were pooled, acetone precipitated and were used to immunize chickens, see Example 11, or submitted for sequence analysis, see Example 10.

EXAMPLE 9

Molecular weight and isoelectric point determination of the four major glycolipid linked proteins from E. Tenella sporozoites

Molecular weights and isoelectric points of the four major glycolipid linked proteins from E. Tenella sporozoites were determined. Molecular weights were determined by analytical SDS-PAGE followed by transfer to nitrocellulose and immunodetection by Western blotting with anti-CRD antibody (as prepared in Example 3). Appropriate molecular weight controls were included. Isoelectric points were determined by Western blotting of two dimensional gels with anti-CRD antibody. The two dimensional gels were run according to the procedure of O'Farrell, J. Biol. Che. 250: 4007-4021 (1975), with modifications similar to the method of Groppi, Mol. Pharmacol. 18: 429-437 (1980).

A detergent rich fraction was prepared from E. Tenella sporozoites (TX114B), Example 5. Aliquots of this fraction were acetone precipitated, and the pellets treated with T. brucei lipase (Example 6). A sample of 50μg of total protein per track was used for analytical SDS PAGE while 100μg of total protein was used for two dimensional gels. The results are shown in Table 2.

TABLE 2

| MOLECULAR WEIGHTS AND ISOELECTRIC POINTS OF THE FOUR MAJOR GLYCOLIPID LINKED PROTEINS FROM E. TENELLA SPOROZOITES | | |
|---|---|---|
| Protein | Molecular Weight (kD ± 2 kD) | Isoelectric Point (PI ± 0.2) |
| 32 | 32 | 4.6 |
| 29 | 29 | 4.8 |
| 26 | 26 | 4.9 |
| 26 | 26 | 5.0 |
| 18 | 18 | 4.8 |
| Note: The 26kD was sometimes resolved into two fused spots with PI's as shown. | | |

EXAMPLE 10

Production of Antibody Against Purified Glycolipid Linked Protein

Fractions from the DEAE HPLC column containing glycolipid linked proteins were applied to 7-20% polyacrylamide Laemmli gels, using 1-5 μg per excised band (see below). After electrophoresis. the gels were stained using Coomassie blue (0.1% in 20% methanol, 7% acetic acid) and destained (20% methanol, 7% acetic acid). Bands of interest were excised, dried by lyophilization and macerated in 0.5 mls of phosphate buffered saline (PBS) by centrifugation (13,000xg, 1 min) through a 0.5ml Eppendorf tube with a 1mm hole in the bottom into a 1.5ml Eppendorf tube. About eight cycles of centrifugation were done. Maceration was completed by multiple passages through a 26 gauge syringe needle. 3-15 μg of protein was used in each 0.5 ml aliquot of PBS; this aliquot was mixed with 0.5 mls of complete Freunds adjuvant and injected subcutaneously into multiple sites on the back of a rabbit. Second and subsequent boosts were given in the same way using incomplete Freunds adjuvant.

The antisera generated against the four major glycolipid linked proteins were used at a dilution of 1:100 in Western blots. The anti-18K glycolipid linked protein (anti 18 kD) antiserum appeared to have the lowest nonspecific binding when tested against sporozoite lysate (E. tenella). This antiserum was further characterized against TX114 top and bottom phase (made from E. tenella sporozoites). Phase separation was done before and after treatment of the lysate with lipase from T. brucei. The antiserum binds to a major protein which comigrates with the 26 kD glycolipid linked protein as well as to an 18 kD protein. In addition, the major reactivity is seen in the bottom fraction when phase separation is performed without lipase treatment. Some of this reactivity moves to the top fraction when lipase treatment is performed prior to phase separation. These changes reflect those seen using the anti-CRD antibody, and is strong evidence that the anti-18K antibody is binding to glycolipid linked proteins.


## EXAMPLE 11


### Protein Sequencing


### Protein Sequencing from Polyacrylamide Gels Using Immobilon  ·

Sequencing of the 32 kD and the 29 kD glycolipid-linked proteins, from Example 7, was performed according the the method of Matsudaira, J. Biol. Chem. 262: 10035-10038 (1987). Briefly, samples (1-5 μg/lane) were loaded onto 7-20% polyacrylamide gels and electrophoresed according to Laemmli. The completed gels were soaked in transfer buffer (10 mM 3-[cyclohexylamino]-1-propanesulfonic acid, Caps, 10% methanol, 5 mM DTT) for five minutes and then electrophoretically transferred to polyvinyldifluoride membranes (PVDF, Immobilon, 0.45 μM pore, Millipore) at 0.5 A for 1.5 hours. After transfer, the membranes were washed in water, 5 minutes, stained with Coomassie blue (0.1% Coomassie blue in 50% methanol) 5 minutes, destained (50% methanol, 10% acetic) 5-10 minutes, and finally washed in water and air dried. The bands of interest were excised from the gel and submitted for protein sequence analysis. Briefly, a polybrene coated glass fiber disc was placed into the cartridge of a 470 sequenator (Applied Biosystems, Foster City, California) equipped with on-line phenylthiohydantoin (PTH) analysis. Three blank cycles were run, according to the manufacturer's instructions, in order to clean the filter. The excised PVDF piece was then placed on top of the glass fiber disc and the sequence analysis was performed.

Silver stained gels showed that some fractions from the anion exchange column contained pure 26 kD glycolipid-linked protein. Several such fractions were pooled, acetone precipitated, and submitted for protein sequence analysis. This was done as described, except that the purified protein was blotted directly onto the polybrene coated filter after the three pre-cycles were completed. The amino terminal sequence of two of the glycolipid linked proteins has been obtained (Table 2). The 29 kD may have a blocked amino terminus.

## TABLE 3

### AMINO TERMINAL SEQUENCE FROM TWO E. TENELLA GLYCOLIPID LINKED PROTEINS

|  | 5 | 10 | 15 |
|---|---|---|---|
| 32 kD | Glu Ser Gly Pro Pro Thr Ala Glu Asp Lys Thr X Ala X Leu Pro |  |  |
|  | Gly     Asn |  |  |

|  | 5 | 10 | 15 |
|---|---|---|---|
| 26 kD | X Ala Pro Ser Ala Ser Lys Lys Thr Thr Glu X Leu Pro X X Asn Ala |  |  |

X = Unidentified residue
Two amino acids shown at a single residue indicates ambiguity at that point.

### EXAMPLE 12

Induction Of Protection To Challenge With E. tenella Glycolipid Linked Protein Immunogens

Broiler pullets were immunized three times via the intramuscular route on days 2, 9 and 16 days of age with samples containing 0.01 to 100 ng of the purified glycolipid linked protein immunogen, from Example 8, in 0.1% SDS, 0.1 ml/bird. Experimental and control birds were challenged at day 23, seven days after the final immunization, with an oral inoculation of from 5 to 30 x $10^3$ sporulated oocysts, an amount sufficient to yield a mean lesion score of at least 2.7 in non-immunized controls at 30 days of age. Seven days after challenge, the chickens were killed and the severity of the lesions in the ceca was determined according to the method of Johnson and Reid, Exp. Parasitol. 28: 30-36 (1970).
Representative examples are shown in the following table.

TABLE 4

| PROTECTION OF CHICKENS AGAINST COCCIDIOSIS WITH GLYCOLIPID LINKED PROTEIN 26 kD | |
|---|---|
| Antigen Dose/Chicken (ng) | Lesion Score |
| 100 | 2.44 |
| 10 | 2.13 |
| 1 | 2.09 |
| 0.1 | 2.00 |
| 0.01 | 2.29 |
| 0 | 2.76 |

These results show that glycolipid linked membrane associated proteins of E. tenella can be used to immunized two-day-old chickens against coccidiosis. Three intramuscular inoculations provide adaquate protection against the disease as indicated by the absense of severe lesion development in immune birds after a normally virulent infection.

**Claims**

1. Substantially pure glycolipid linked membrane associated protein immunogens obtained from Eimeria species oocysts, sporulated oocysts and sporozoites with said immunogens being capable of immunizing poultry against coccidiosis.

2. The substantially pure Eimeria immunogens of claim 1 wherein the glycolipid portion of the immunogen will bind to anti-CRD antibody after lipase treatment.

3. The Eimeria glycolipid linked membrane associated protein immunogen of claim 1 wherein the species is selected from a group of Eimeria species consisting of E. acervulina, E. mivati, E. mitis, E. preacox, E. hagani, E.nactrix, E. maxima, E. burnetti and E. tenella.

4. The Eimeria glycolipid linked membrane associated protein immunogen of claim 3 wherein the Eimeria species is E. tenella.

5. The protein immunogens of claim 1 wherein said proteins are substantially free of the associated glycolipid membrane anchor.

6. A protein immunogen of claim 5 wherein the protein has a molecular weight of about 32,000 daltons and an isoelectric point of about 4.6

7. A protein immunogen of claim 5 wherein the protein has a molecular weight of about 29,000 daltons and an isoelectric point of about 4.8.

8. A protein immunogen of claim 5 wherein the protein has a molecular weight of about 26,000 daltons and an isoelectric point of about 5.0.

9. A protein immunogen of claim 5 wherein the protein has a molecular weight of about 18,000 daltons and an isoelectric point of about 4.8.

10. An Eimeria tenella protein immunogen comprising at least the following amino acid sequence:

```
                              5                        10
    X Ala Pro Ser Ala Ser Lys Lys Thr Thr Glu X
          15
    Leu Pro X X Asn Ala
```

and any microheterogeneous or subunit immunogen forms thereof.

11. An Eimeria tenella protein immunogen comprising at least the following amino acid sequence:

```
                                              10
    Glu Ser Gly Pro Pro Thr Ala Glu Asp Lys Thr
    Gly     Asn
            15
    X Ala X Leu Pro
```

and any microheterogeneous or subunit immunogen forms thereof.

12. A method of preparing the glycolipid linked membrane associated protein immunogens of claim 1 comprising:

    a. preparing lysates of unsporulated oocysts, sporulated oocytst and sporozoites;
    b. subjecting the lysates to Triton X114;
    c. collecting the detergent rich phase;
    d. precipitation of the detergent rich phase protein; and

e. separation by anion exchange chromatography.

13. An <u>Eimeria</u> immunogen composition comprising an immunologically effective amount of one or more of the inmunogens as claimed in any one of claims 1, or 6 - 11 in a physiologically acceptable medium.

14. The use of the immunogen as claimed in anyone of claims 1, or 6 - 11, for the preparation of a composition useful for immunizing poultry against coccidiosis.

16